# EUROPEAN PATENT SPECIFICATION

(11) **EP 0 575 370 B1**
(45) Date of publication and mention of the grant of the patent: **24.04.1996**
(21) Application number: 92905562.2
(22) Date of filing: 27.02.1992
(51) Int. Cl.: C07D 223/28, A61K 31/645

(54) **PROCESS FOR THE PURIFICATION OF LOFEPRAMINE BY CRYSTALLISATION**
VERFAHREN ZUR REINIGUNG VON LOFEPRAMIN DURCH KRISTALLISATION
PROCEDE DE PURIFICATION DE LA LOFEPRAMINE PAR CRISTALLISATION

(30) Priority: 13.03.1991 DE 4108119
(43) Date of publication of application: 29.12.1993
(73) Proprietor: SCHULER, Wilhelm, D-61348 Bad Homburg (DE); JELLALABAD LTD., Arklow Co. Wicklow (IE)
(72) Inventor: SCHULER, Wilhelm, D-6380 Bad Homburg (DE); LYONS, Richard, Arklow, Co. Wicklow (IE)
(74) Representative: Sternagel, Hans-Günther, Dr.
(86) International application number: EP9200428
(87) International publication number: WO9219599

(56) References cited:
- FR-A- 2 316 234
- JOURNAL OF CRYSTALLOGRAPHIC AND SPECTROSCOPIC RESEARCH, vol. 17, no. 3, 1987, NEW-YORK, US, pages 281-293; G. BANDOLI ET AL.: 'Crystal structure of two tricyclic antidepressant drugs: Lofepramine hydrochloride and dothiepin hydrochloride'

## Description

The present invention refers to a process for the preparation of purified, storage stable 4'-chloro-2[[3-(10,11)-dihydro-5H-dibenz(b,f)azepin-5-yl)propyl]methylamino]acetophenone (Lofepramin) and its salts by using a specific organic solvent for the crystallisation of the crude Lofepramine and its use the preparation of pharmaceutical compositions, particularly to the compound which has the following structure: 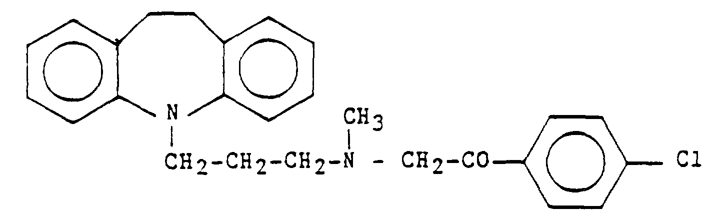 Hereinafter this compound will be referred to as Lofepramine (INN).

Lofepramine hydrochloride has proved clinically effective for treatment of mental depressions.

A process for the production of Lofepramine hydrochloride is disclosed in GB-A.1,177,525. E. Eriksoo and 0. Rohte described in Arzneimittelforschung 20, 1561-1569 (1970) a preferred method of synthesis. Thereby 10,11-dihydro-5-[3-(methylamino)propyl]-5H-dibenz(b,f)azepin (INN: Desipramine is reacted with 2-bromo-4'-chloroacetophenone, dissolved in benzene, in the presence of an aqueous phase containing sodium hydrogen carbonate (two-phase system).

This procedure, however, presents difficulties as mentioned in DE-A-26 28 558, page 2. The Lofepramine base remaining after distilling off benzene is a brown colour and has an oily, ointmentlike consistency, from which only a slightly greenish, or grey Lofepramine hydrochloride can be produced.

A further method for the preparation of Lofepramine is described in DE-B-26 28 558. Accordingly, Desipramine is reacted with 2-bromo-4'-chloroacetonphenone in a two-phase system, in which special esters, or ketones are used as organic solvents. The Lofepramine base that precipitates is filtered, washed with different solvents and then converted to Lofepramine hydrochloride.

Lofepramine hydrochloride prepared according to this method decomposes on storing. After a few days it begins to change colour. The faint yellow salt becomes a darker yellow and after further days turns brown. The shelf life of the salt, can be improved if is is stored in air-tight containers and protected from light. But, even Lofepramine hydrochloride stored in this way has to be utilized soon because of its instability. In GB-A-1,498,857 there is described that therapeutic compositions (e.g. tablets) which contain Lofepramine hydrochloride decompose. Two decomposition products are mentioned. It is mentioned that it has, however, been impossible to determine the exact nature of this decomposition. This above mentioned British patent stabilizes Lofepramine hydrochloride containing solid therapeutic compositions (e.g. tablets) by adding ascorbic acid.

All the known methods do not result in a sufficient storable product and the yields are low.

It is the object of the present invention to provide a method for purifying Lofepramine or its salts having improved yield and resulting in a storage stable product.

This object is attained by a method for the preparation of a purified, storage stable Lofepramine of the structure 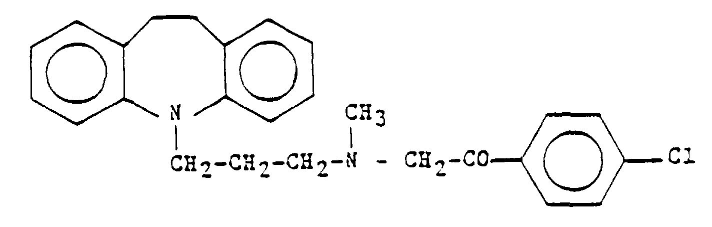 and/or its salts, by reacting Desipramine base and 2-bromo-4'-chloroacetophenone in a two-phase system, by known procedures, separating in the phases and removing the organic solvent from the organic phase, dissolving the residue of the crude Lofepramine in an organic solvent and crystallizing a purified Lofepramine from the solution, characterized by using methyl-tertiary-butylether for the purification and crystallization and, if desired, converting the Lofepramine base thus obtained to its salts.

The purified, storage stable crystalline product can, if desired, furthermore be stabilized by suspending the Lofepramine salt in methyl-tertiary-butylether, then adding an aqueous solution of tartaric acid to said suspension and removing the water by azeotropic destillation and crystallizing the Lofepramine salt from the solvent.

The purified, storage stable Lofepramine or its salts obtained by the method according to the present invention can be used for the preparation fo pharmaceutical compositions.

It was found that Lofepramine base can be produced in a faint yellow, crystalline form in good yield, when the brown, oily, ointment-like Lofepramine base prepared by the known procedure of E.Eriksoo and 0. Rohte is crystallized using methyl-tert-butylether.

The known procedure of E. Eriksoo and 0. Rohte involves the reation between Desipramine base and 2-bromo-4'chloroacetophenone in a two-phase system (e.g. benzene/ water) according to Arzneimittelforschung 20, 1561-1569 (1970). According to this procedure the organic phase containing the formed Lofepramine base is separated from the water phase and the organic solvent removed (e.g. vacuum distillation).

The methyl-tert-butylether used according to this invention, proved to be an excellent solvent, producing pure Lofepramine base. The treatment of Lofepramine base occurs at temperatures above room temperature (20°) and up to the boiling point of the ether, for example at 40°C to 50°C, preferably at 45° to 50°C for 10 to 40 minutes, preferably for 25 to 30 minutes. For one part by weight of crude Lofepramine base 4 to 9 parts by weight, preferably 5 to 7 parts by weight of methyl-tert-butylether are used.

The crystallization occurs in a way that the bulk of the crude base precipitates as a crystalline solid form the hot solution (temperature for example 30° to 40°C) during this operation, the remainder precipitates when cooling slowly to room temperature. The Lofepramine base obtained can be converted to its salts by known procedures.

This conversion into the corresponding salts is effected by dissolving the clean Lofepramine base, prepared according to the present invention, in a common organic solvent, for example, a saturated aliphatic liquid ketone with 3 to 6 carbon atoms such as acetone, methylethylketone, 2-pentanone, 3-pentanone, 3-methyl-2-butanone, 2-hexanone, 3-hexanone, ethylisopropylketone, methylisopropylketone, 2,2-dimethyl-3-butanone, preferably methylethylketone, and by adding to this solution the corresponding acid, or a solution of the acid in a common organic solvent.

This salt formation can be carried out at temperatures between 30° to 100°C, preferably at 40°C to 70°C, usually one part by weight of Lofepramine base is dissolved in 2 to 5 parts, preferably 3 to 4 parts by weight of the organic solvent.

For instance, in making Lofepramine hydrochloride, one part by weight of Lofepramine is dissolved in 3 to 5 parts, preferably in 3 to 4 parts by weight of methylethylketone and treated with hydrogen chloride.

The stability of Lofepramine hydrochloride prepared according to the present invention during storage and when manufacturing tablets is improved in comparison to the stability of the prior art products. The storage stability is sufficient for most applications. It was found that Lofepramine salts, particularly Lofepramine hydrochloride, can be stabilised remarkably when they are treated with tartaric acid. The Lofepramine salts are typically suspended in a small amount of methyl-tertiary-butylether, and a small amount of tartaric acid dissolved in water is added. The water is distilled off azeotropically together with some methyl-tert-butylether. The distillation is continued until all the water is removed from the methyl-tert-butylether/water mixture.

When the azeotropic distillation is finished the reaction mixture is allowed to cool, with stirring, to room temperature, the stabilized Lofepramine salt crystallizes.

For treatment with tartaric acid it is typical to use for one part by weight of Lofepramine salt 0.005 to 0.02, preferably 0.01 to 0.02 parts by weight of tartaric acid. The tartaric acid is used as a solution in water, for example, as a 30-60%, preferably a 50-60% water solution (weight/volume).

For the suspension of Lofepramine salts in methyl-tert-butylether, for one part by weight of Lofepramine salt (e.g. hydrochloride) 3 to 6, preferably 4 parts by weight of methyl-tert-butylether are used.

The treatment with tartaric acid is carried out, for example, by adding the water solution of tartaric acid at 10° to 50°C, preferably at 20°C to 30°C to the suspension of the Lofepramine salts and keeping the mixture at this temperature for 15 to 20 mins., then heating to the boiling point and distilling off the water azeotropically.

Lofepramine salts treated by this method are stable for storing and, for example, manufacturing tablets and coated pills.

For example, when a sample of untreated Lofepramine hydrochloride and a second sample of Lofepramine hydrochloride which is treated with tartaric acid are stored in the open air, the untreated sample turns notably yellow after 1 to 3 days and brownish yellow after further days storing. Thin layer chromatography shows two distinct spots which indicate decomposition products of Lofepramine, which are undesired in therapeutic compositions.

The invention will now be described in the following examples.

### Example 1

25g of Desipramine base (10,11-dihydro-5-[3-(methylamino)propyl]-5H-dibenz(b,f)azepine]) and 2-bromo-4'-chloroacetophenone are added to 140 ml toluene. A solution of 10.0 g sodiumbicarbonate and 1.0 g sodium sulfite dissolved in 75 ml water is added and the mixture stirred for 3.5 hours at room temperature. The aqueous phase is separated and discarded. The organic phase is washed with 50 ml water. The organic phase is then evaporated to dryness under vacuum and a brownish, oily, ointment-like Lofepramine base remains. 200 ml of methyl-tert-butylether are added at 50°C to this residue and the mixture refluxed for 30 mins. Lofepramine base precipitates as a light yellow powder. Approximately 150 ml of methyl-tert-butylether are distilled off. Then the mixture is allowed to cool overnight. The product is filtered, washed and dried. 32,2 g (83%) of Lofepramine base are obtained as a faint yellow, crystalline powder.

According to normal procedure this Lofepramine base is dissolved in a fivefold quantity of methylethylketone and converted to Lofepramine hydrochloride using the equivalent amount of gaseous hydrogenchloride.

### Comparative Example 1

The Lofepramine base was prepared according to Example 1 but 200 ml acetone instead of methyl-tert-butylether are added to the oily residue after removal of the solvent and the mixture was refluxed for 30 minutes. From the cooled situation 3,2 g purified Lofepramine base could be crystallized. This is a yield of 8.6 % of the theoretical yield.

### Comparative Example 1a

Comparative Example 1 was repeated, but 200 ml methanol were used instead of acetone. The yield of the purified product was 13,6 g (35%) of a yellow coloured product.

### Example 2

40.0 g of Lofepramine hydrochloride are suspended in 160 ml methyl-tert-butylether and to this is added a solution of 0.7g of tartaric acid in 1.3 ml water. The mixture is heated to reflux and the water is removed by azeotropic distillation. The reaction mixture is cooled to room temperature with stirring. The product is filtered, washed with methyl-tert-butylether and dried. 37.24 g (94%) of light yellow Lofepramine hydrochloride crystals are obtained.

This layer chromatography shows that this treated Lofepramine hydrochloride, when stored in the open air, is stable compared to an untreated sample which showed distinct spots that point to decomposition products, or storing for four days.

## Claims

1. A process for the preparation of a purified, storage stable Lofepramine of the structure 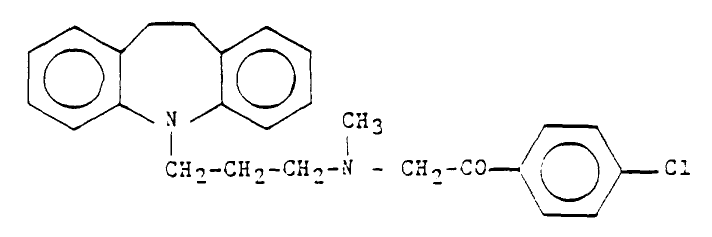 and/or its salts, by reacting Desipramine base and 2-bromo-4'-chloroacetophenone in a two-phase system, by known procedures, separating in the phases and removing the organic solvent from the organic phase, dissolving the residue of the crude Lofepramine in an organic solvent and crystallizing a purified Lofepramine from the solution,
**characterized by**
using methyl-tertiary-butylether for the purification and crystallization and, if desired, converting the Lofepramine base thus obtained to its salts.

2. The process of claim 1
**characterized by**
using 6 parts by weight of methyl-tertiary-butylether for one part by weight of the crude Lofepramine base.

3. The process of claim 1 or 2
**characterized by**
treating the crude Lofepramine base with methyl-tertiary-butylether at a temperature between 45°C and 50°C.

4. The process of any of the preceding claims
**characterized by**
converting the Lofepramine base by known methods into a salt of a pharmacologically compatible acid.

5. The process of claim 4
**characterized by**
converting the Lofepramine base with hydrochloric acid into the hydrochloride salt.

6. The process of claims 4 or 5
**characterized by**
suspending the purified Lofepramine salt in methyl-tertiary-butylether, adding an aqueous solution of tartaric acid to said suspension and removing the water by azeotropic distillation and crystallizing Lofepramine salt.

7. The process of claim 6
**characterized by**
using form 0.005 to 0.02 parts by weight of tartaric acid for one part by weight of Lofepramine salt.

8. The process of claims 6 or 7
**characterized by**
using from 3 to 6 parts by weight methyl-tertiary-butylether for one part by weight of Lofepramine salt.

## Patentansprüche

1. Verfahren zur Herstellung eines gereinigten, lagerstabilen Lofepramins der Formel 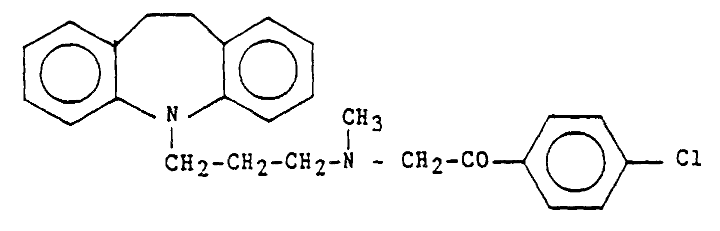 und/oder seiner Salze durch Umsetzen von Desipraminbase und 2-Brom-4'-chloracetophenon in einem Zwei-Phasen-System mittels bekannter Verfahren, Aufteilen in die Phasen und Entfernen des organischen Lösemittels aus der organischen Phase, Auflösen des Rückstandes aus rohem Lofepramin in einem organischen Lösemittel und Kristalli. sieren eines gereinigten Lofepramins aus der Lösung,
**gekennzeichnet durch**
Verwendung von Methyl-tertiär-butylether für die Reinigung und Kristallisierung und, falls erwünscht, Umwandeln der auf diese Weise erhaltenen Base in ihre Salze.

2. Verfahren nach Anspruch 1,
**dadurch gekennzeichnet,**
daß 6 Gewichtsteile von Methyl-tertiär-butylether für 1 Gewichtsteil der rohen Lofepraminbase verwendet werden.

3. Verfahren nach Anspruch 1 oder 2,
**dadurch gekennzeichnet,**
daß das Behandeln der rohen Lofepraminbase mit Methyl-tertiär-butylether bei einer Temperatur zwischen 45°C und 50°C ausgeführt wird.

4. Verfahren nach einem der vorstehenden Ansprüche,
**dadurch gekennzeichnet,**
daß die Lofepraminbase mittels bekannter Methoden in ein Salz einer pharmakologisch verträglichen Säure umgewandelt wird.

5. Verfahren nach Anspruch 4,
**dadurch gekennzeichnet,**
daß die Lofepraminbase mittels Salzsäure in das Hydrochloridsalz umgewandelt wird.

6. Verfahren nach Ansprüchen 4 oder 5,
**gekennzeichnet durch**
Suspendieren des gereinigten Lofepraminsalzes in Methyl-tertiär-butylether, Zugeben einer wäßrigen Lösung von Weinsäure zur Suspension und Entfernen des Wassers durch azeotrope Destillation und Kristallisieren des Lofepraminsalzes.

7. Verfahren nach Anspruch 6,
**dadurch gekennzeichnet,**
daß 0,005 bis 0,02 Gewichtsteile Weinsäure für 1 Gewichtsteil Lofrepraminsalz verwendet werden.

8. Verfahren nach Ansprüchen 6 oder 7,
**dadurch gekennzeichnet,**
daß 3 bis 6 Gewichtsteile Methyl-tertiär-butylether für 1 Gewichtsteil Lofepraminsalz verwendet werden.

## Revendications

1. Procédé pour la préparation de Lofepramine stable au stockage, purifiée, de structure 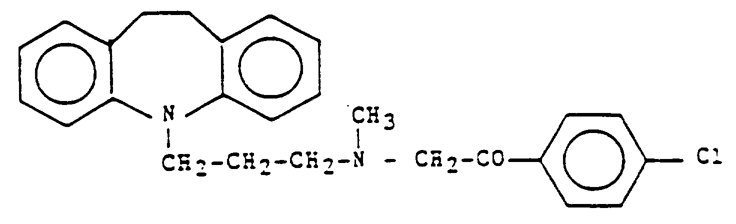 et/ou de ses sels, en faisant réagir la Désipramine base et la 2-bromo-4'-chloroacétophénone dans un système à deux phases, à l'aide de procédés connus, en séparant dans les phases et en éliminant le solvant organique de la phase organique, en dissolvant le résidu de Lofepramine brute dans un solvant organique et en cristallisant une Lofepramine purifiée à partir de la solution,
caractérisé par l'utilisation de méthyl-tert-butyléther pour la purification et la cristallisation et, si souhaité, en convertissant la Lofepramine base ainsi obtenue en ses sels.

2. Procédé selon la revendication 1, caractérisé par l'utilisation de 6 parties en poids de méthyl-tert-butyléther pour une partie en poids de Lofepramine base brute.

3. Procédé selon l'une des revendications 1 ou 2, caractérisé par le traitement de la Lofepramine base brute avec le méthyl-tert-butyléther à une température comprise entre 450C et 500C.

4. Procédé selon l'une des revendications précédentes, caractérisé par la conversion de la Lofepramine base, à l'aide de procédés connus, en un sel d'un acide compatible sur le plan pharmacologique.

5. Procédé selon la revendication 4, caractérisé par la conversion de la Lofepramine base avec de l'acide chlorhydrique en son chlorhydrate.

6. Procédé selon l'une des revendications 4 ou 5, caractérisé par la mise en suspension du sel de Lofepramine purifié dans le méthyl-tert-butyléther, par l'addition d'une solution aqueuse d'acide tartrique à ladite suspension, et par l'élimination de l'eau à l'aide d'une distillation azéotrope et par la cristallisation du sel de Lofepramine.

7. procédé selon la revendication 6, caractérisé par l'utilisation de 0,005 à 0,02 partie en poids d'acide tartrique pour une partie en poids de sel de Lofepramine.

8. Procédé selon l'une des revendications 6 ou 7, caractérisé par l'utilisation de 3 à 6 parties en poids de méthyl-tert-butyléther pour une partie en poids de sel de Lofepramine.
